# NEUE EUROPÄISCHE PATENTSCHRIFT

(11) **EP 0 708 813 B2**
(45) Veröffentlichungstag und Bekanntmachung des Hinweises auf die Entscheidung über den Einspruch: **07.02.2001**
(45) Hinweis auf die Patenterteilung: 18.12.1996
(21) Anmeldenummer: 94921518.0
(22) Anmeldetag: 08.07.1994
(51) Int. Cl.: C11C 3/04, C07C 67/03, C07C 69/24, C07C 69/52

(54) **VERFAHREN ZUR HERSTELLUNG VON FETTSÄUREALKYLESTERN**
PROCESS FOR PREPARING FATTY ACID ALKYL ESTERS
PROCEDE DE PREPARATION D'ESTERS D'ALKYLE D'ACIDE GRAS

(30) Priorität: 14.07.1993 AT 139993
(43) Veröffentlichungstag der Anmeldung: 01.05.1996
(73) Patentinhaber: BDI Anlagenbau Gesellschaft mbH, 8073 Feldkirchen (AT); Mittelbach, Martin, A-8010 Graz (AT); Koncar, Michael, A-8501 Lieboch (AT)
(72) Erfinder: MITTELBACH, Martin, A-8010 Graz (AT); KONCAR, Michael, A-8501 Lieboch (AT)
(74) Vertreter: Schwarz, Albin, Dr.
(86) Internationale Anmeldenummer: AT9400088
(87) Internationale Veröffentlichungsnummer: WO9502661

(56) Entgegenhaltungen:
- EP-A- 0 131 991
- EP-A- 0 591 019
- WO-A-93/09212
- AT-A- 392 977
- FR-A- 2 696 185
- OLEAGINEUX, Bd.40, Nr.3, 1985, FR Seiten 147 - 151 R.C.A. LAGO ET AL. 'Extraction and transesterification of vegetable oils with ethanol'

## Beschreibung

Die Erfindung betrifft ein Verfahren zur Herstellung vpn Fettsäurealkylestem durch Umesterung, Insbesondere katalytische Umesterung, von Triglyceriden, wobei aus einem Reaktionsgemisch, in dem die Umesterung vorgenommen wird, eine Esterphase und eine Fettsäuren, Fettsäuresalze und/oder andere Fettsäureverbindungen enthaltende Glycerinphase gebildet werden, die voneinander getrennt werden.

Unter Umesterung ist die Alkoholyse von Triglyceriden zu verstehen, also die Umsetzung mit Alkoholen, insbesondere Methanol und Ethanol, wobei über die Zwischenprodukte Di- und Monoglyceride die Monoester der Fettsäuren sowie Glycerin entstehen.

Fettsäureester, insbesondere die Methylester, sind wichtige Zwischenprodukte in der Oleochemie. Allein in Europa werden jährlich 200.000 Tonnen Pflanzenölmethylester als Rohstoffe vor allem für Tenside hergestellt. Daneben gewinnt der Fettsäuremethylester als Dieselersatzkraftstoff immer mehr an Bedeutung.

Als Katalysatoren für die Umesterung können basische Katalysatoren (Alkalihydroxide, -alkoholate, -oxide, -carbonate, Anionenaustauscher), saure Katalysatoren (Mineralsäuren, p-Toluolsulfonsäure; Bortrifluorid, Kationenaustauscher) und Enzyme (Lipasen) verwendet werden. Bevorzugt werden heute im Reaktionsgemisch lösliche Katalysatoren verwendet. Diese bilden ein homogenes Gemisch und gewährleisten schnelle Umsatzraten und milde Reaktionsbedingungen. Die am häufigsten verwendeten homogenen Katalysatoren sind Natrium- und Kaliumhydroxid sowie Natriummethylat, welche in Alkohol gelöst dem Pflanzenöl zugemischt werden. Ein derartiges Verfahren ist aus der AT-B 386 222 bekannt. Die saure Katalyse erfordert höhere Reaktionstemperaturen und -drücke und eine aufwendigere Reaktionsführung. Eine saure Umesterung ist aus der FR-A - 85 02340 bekannt.

Die Umesterung mit basischer Katalyse wird ohne Verwendung eines Lösungsmittels durchgeführt. Die Reaktion beginnt mit einem Zweiphasensystem aus Triglycerid und Alkohol, mit zunehmendem Reaktionsfortschritt und Bildung von Ester entsteht aber eine homogene Phase, welche durch Bildung und Ausscheidung von Glycerin wiederum zweiphasig wird.

Bei der Alkoholyse von Triglyceriden zur Herstellung von Estem der Fettsäuren mit einwertigen Alkoholen fällt als Nebenprodukt eine glycerinreiche Phase an. Diese Phase enthält ferner Fettsäuren, Fettsäuresalze und Fettsäureester. Um diese Fettsäureverbindungen aus der Glycerinphase abzutrennen, wird sie in der Regel mit Säuren behandelt. Durch diese Behandlung werden die Fettsäuren aus den Fettsäuresalzen freigesetzt. Die Fettsäuren sowie die Fettsäureester selbst sind nicht mit Glycerin mischbar und setzen sich daher als eigene Phase von der Glycerinphase ab. Diese Phase wird als Fettsäurephase bezeichnet.

Eine sinnvolle Verwendung dieser Fettsäurephase ist bis heute nicht aufgezeigt worden, und diese Phase wurde im Stand der Technik in der Regel als Verlust des Umesterungsverfahrens in Kauf genommen. Dieser Verlust wirkt sich insbesondere dann ungünstig aus, wenn das Verfahren in großtechnischem Maßstab durchgeführt wird, wo bereits eine Verbesserung in der Größenordnung von 1-2 % die Wirtschaftlichkeit des Verfahrens entscheidend erhöht.

Die Erfindung stellt sich die Aufgabe, ein verbessertes Verfahren zur Herstellung von Fettsäurealkylestem durch Umesterung, insbesondere katalytische Umesterung, von Triglyceriden zur Verfügung zu stellen.

Das erfindungsgemäße Verfahren ist dadurch gekennzeichnet, daß man die Fettsäuren, die Fettsäuresalze und/oderanderen Fettsäureverbindungen aus der Glycerinphase abgetrennt, mit einem Alkohol aus der Gruppe Methanol, Ethanol, Propanol, i-Propanol, Butanol, sek.-Butanol, Pentanol, Hexanol, Heptanol und Octanol verestert und einem anderen Reaktionsgemisch, in dem eine weitere Umesterung vorgenommen wird, wieder zuführt.

Die Veresterung kann nach bekannten Verfahren durchgeführt werden, wobei eine Veresterung mit einem sauren Katalysator besonders zweckmäßig ist. Beispielhaft wird eine saure Katalyse mit Schwefelsäure, Paratoluolsulfonsäure oder mit lonenaustauschem in H⁺-Form genannt.

Eine Veresterung freier Fettsäuren ist aus der EP-A - 0 184 740 bekannt Allerdings handelt es sich bei diesem Verfahren um eine Vorveresterung freier Fettsäuren, die in manchen natürlich vorkommenden Fetten und Ölen enthalten sind. Ausgehend von natürlichen Fetten und Ölen, die freie Fettsäuren enthalten, können Fettsäuremethylester dadurch erhalten werden, daß man die im Ausgangsmaterial vorhandenen freien Fettsäuren in Gegenwart von sauren Katalysatoren zuerst mit überschüssigem Methanol verestert, eine den Katalysator enthaltende Alkoholphase abtrennt, die verbleibende Ölphase (Triglyceridphase) mit einem Glycerin-Methanol-Gemisch extrahiert und die behandelte ölphase einer alkalikatalysierten Umesterung mit Methanol unterwirft. Es hat sich jedoch gezeigt, daß auch bei der Alkoholyse der Triglyceride Fettsäureverbindungen mit der Glycerinphase aus dem Umesterungsprozeß abgezogen werden, wodurch die Ausbeute sinkt. Durch die erfindungsgemäß vorgenommene Veresterung der nach Umesterung und nach Aufbereitung der Glycerinphase gewonnenen Fettsäurephase werden nicht nur die im verarbeiteten Triglycerid vorhandenen freien Fettsäuren erfaßt und zu Fettsäureester verarbeitet, sondern es können auch die bei der Umesterung entstandenen Fettsäureverbindungen wieder zurückgewonnen werden.

Aus der EP-B - 0 192 035 ist ebenfalls ein Verfahren zur Vorveresterung freier Fettsäuren in Rohfetten und/ oder -ölen bekannt. Bei diesem Verfahren werden zur Senkung des Gehalts an freien Säuren in Fetten und/ oder Ölen diese mit einem niederen Monoalkohol in Gegenwart saurer Veresterungskatalysatoren behandelt, wobei als Katalysator feste Kationenaustauscherharze in saurer Form eingesetzt werden und die Entfernung des Reaktionswassers nach der Abtrennung des Reaktionsgemisches von dem Kationenaustauscherharz vorgenommen wird.

Das erfindungsgemäße Verfahren bietet insbesondere bei der alkalisch katalysierten Umesterung von Triglyceriden Vorteile. Bei dieser Art der Umesterung ist es trotz saurer Veresterung möglich, das saure Rohprodukt direkt und ohne Abtrennung des sauren Katalysators wieder einem Umesterungsprozeß zuzuführen. Auf Grund der Mengenverhältnisse zwischen saurem Katalysator der Veresterung und alkalischem Katalysator der Umesterung kommt es nur zu unwesentlichen Änderungen des pH-Wertes im Umesterungsprozeß. Der saure Katalysator der Veresterung wird mit der Glycerinphase aus dem Prozeß ausgeschleust. Eventuell noch nicht veresterte freie Fettsäuren werden ebenfalls als Seifen mit der Glycerinphase abgezogen. Die bei der Veresterung der Fettsäurephase entstandenen Ester bleiben in der Esterphase. Auf diese Weise kann die Ausbeute des Umesterungsverfahrens wesentlich erhöht werden.

Eine besonders bevorzugte Ausführungsform des erfindungsgemäßen Verfahrens besteht darin, daß die veresterten Fettsäuren, Fettsäuresalze und/oder anderen Fettsäureverbindungen dem anderen bzw. dem weiteren Reaktionsgemisch zu einem Zeitpunkt zugegeben werden, an dem die weitere Umesterung im wesentlichen beendet ist, sich aber die Esterphase und die Glycerinphase der anderen Umesterung noch nicht voneinander getrennt haben.

Es hat sich weiters gezeigt, daß sich das erfindungsgemäße Verfahren am besten so durchführen läßt, daß die Umesterung in mehreren aufeinanderfolgenden Stufen durchgeführt wird, wobei in jeder Stufe eine Fettsäurephase gebildet wird, die verestert und einer nachfolgenden Umesterungsstufe zugegeben wird.

Eine weitere, beonders zweckmäßige Variante des erfindungsgemäßen Verfahrens besteht darin, daß die Umesterung in mehreren aufeinanderfolgenden Stufen durchgeführt und die in den Stufen jeweils anfallenden Glycerinphasen abgetrennt und vereinigt werden, und daß aus den vereinigten Glycerinphasen eine Fettsäurephase gebildet wird, welche verestert und dem anderen Reaktionsgemisch, in dem die weitere Umesterung vorgenommen wird, zugeführt wird.

Als Triglyceride können im erfindungsgemäßen Verfahren sämtliche Fette und Öle pflanzlichen und tierischen Ursprungs umgeestert werden. Beispielhaft werden genannt: Rapsöl, Sojabohnenöl, Sonnenblumenöl, Talg, Palmöl und Palmfett, Ricinusöl, Kokosöl und Kokosfett, Olivenöl, Erdnußöl, Saffloräl, Leinöl, Purgiernußöl, Baumwollsaatöl, Reisöl, Schweinefett. Das erfindungsgemäße Verfahren eignet sich für eine Vielzahl von Ausgangsprodukten verschiedenster Qualität, die Palette reicht von den Pflanzenölen in Speiseölqualität über unraffinierte Öle bis hin zu tierischen Fetten oder Fettabfällen, wie gebrauchte Hydrauliköle auf Fettbasis, sowie gebrauchten Speiseölen.

Als Triglyceride eignen sich insbesondere Rapsöl, Sojabohnenöl und Talg sehr gut, wobei durch die Umesterung Rapsölfettsäuremethylester, Sojabohnenfettsäuremethylester und Talgfettsäuremethylester hergestellt werden, sofern als Alkohol Methanol eingesetzt wird.

Es hat sich gezeigt, daß sich das erfindungsgemäße Verfahren inbesondere zum Umestern von Fetten und/oder ölen tierischer oder pflanzlicher Herkunft eignet, die freie Fettsäuren enthalten. Eine weitere bevorzugte Variante des erfindungsgemäßen Verfahrens besteht somit darin, daß als pflanzliches Öl Palmöl bzw. Palmfett eingesetzt wird. Palmöl kann bis zu 15% freie Fettsäuren enthalten.

Eine aus abfallwirtschaftlichen Gründen interessante Variante des erfindungsgemäßen Verfahrens besteht darin, daß als Triglycerid Abfallöl und/oder Abfallfett, wie gebrauchtes Speiseöl und/oder Frittierfett oder gebrauchte Hydrauliköle auf Fettbasis eingesetzt werden.

Als Alkohole für die Umesterung und/oder die Veresterung eignen sich sehr gut Alkohole mit 1 oder 2 Kohlenstoffatomen.

Die vorliegende Erfindung wird mit dem nachfolgenden Ausführungsbeispiel in einer bevorzugten Ausführungsform noch näher beschrieben.

### Beispiel

31,4 g einer Fettsäurephase, welche aus dem in der AT-B 386.222 beschriebenen alkalischen Umesterungsverfahren zur Herstellung von Rapsölmethylester aus Rapsöl durch Neutralisation der entstandenen Glycerinphase gewonnen wurde und einen Methanolgehalt von 8,1 Masse% aufwies, wurde mit 3,3 g Methanol und 0,3 g konzentrierter Schwefelsäure vermischt und unter Rückfluß bei einer Temperatur von ca. 85°C 2 Stunden gekocht. Durch die Veresterung konnte der Fettsäuregehalt der Fettsäurephase von 50 Masse% auf 12,5 Masse% verringert werden. Das erhaltene Gemisch aus Estern, Fettsäuren und Schwefelsäure wurde einem Reaktionsgemisch einer weiteren Umesterung nach Beendigung der Reaktion (= Alkoholyse), aber noch vor der Phasentrennung, zugeführt. Diese andere Umesterung wurde unter den gleichen Bedingungen wie die erste Umesterung durchgeführt.

Durch den Zusatz der veresterten Fettsäurephase konnte die Ausbeute an Methylester gesteigert werden. Bei der Umesterung mit Veresterung und Rückführung der Fettsäurephase konnten aus 100 g Rapsöl 100 g Methylester (RME) gewonnen werden. Ohne Rückführung der Fettsäurephase lag die Ausbeute bei 97 g RME pro 100 g Rapsöl.

Unabhängig von der Reaktionsführung - ob mit oder ohne Rückführung der veresterten Fettsäurephase - hatte der erhaltene Methylester immer die gewünschte Qualität.

## Patentansprüche

1. Verfahren zur Herstellung von Fettsäurealkylestern durch Umesterung, insbesondere katalytische Umesterung, von Triglyceriden, wobei aus einem Reaktionsgemisch, in dem die Umesterung vorgenommen wird, eine Esterphase und eine Fettsäuren, Fettsäuresalze und/oder andere F.ettsäureverbindungen enthaltende Glycerinphase gebildet werden, die voneinander getrennt werden, dadurch gekennzeichnet, daß die Fettsäuren, die Fettsäuresalze und/oder anderen Fettsäureverbindungen aus der Glycerinphase abgetrennt, mit einem Alkohol aus der Gruppe Methanol, Ethanol, Propanol, i-Propanol, Butanol, sek.-Butanol, Pentanol, Hexanol, Heptanol und Octanol verestert und einem anderen Reaktionsgemisch, in dem eine weitere Umesterung vorgenommen wird, wieder zugeführt werden.

2. Verfahren nach Anspruch 1, dadurch gekennzeichnet, daß die Veresterung mit einem sauren Katalysator durchgeführt wird.

3. Verfahren nach Anspruch 2, dadurch gekennzeichnet, daß das nach Veresterung mit einem sauren Katalysator erhaltene Rohprodukt ohne weitere Behandlung dem anderen Reaktionsgemisch zugegeben wird.

4. Verfahren nach einem oder mehreren der Ansprüche 1 bis 3, dadurch gekennzeichnet, daß die Umesterung unter alkalischer Katalyse durchgeführt wird.

5. Verfahren nach einem oder mehreren der Ansprüche 1 bis 4, dadurch gekennzeichnet, daß die veresterten Fettsäuren, Fettsäuresalze und/oder anderen Fettsäureverbindungen dem anderen Reaktionsgemisch zu einem Zeitpunkt zugegeben werden, an dem die weitere Umesterung im wesentlichen beendet ist, sich aber die Esterphase und die Glycerinphase der anderen Umesterung noch nicht voneinander getrennt haben.

6. Verfahren nach einem oder mehreren der Ansprüche 1 bis 5, dadurch gekennzeichnet, daß die Umesterung in mehreren aufeinanderfolgenden Stufen durchgeführt wird, wobei in jeder Stufe eine Fettsäurephase gebildet wird, die verestert und einer nachfolgenden Umesterungsstufe zugegeben wird.

7. Verfahren nach einem oder mehreren der Ansprüche 1 bis 5, dadurch gekennzeichnet, daß die Umesterung in mehreren aufeinanderfolgenden Stufen durchgeführt und die in den Stufen jeweils anfallenden Glycerinphasen abgetrennt und vereinigt werden, und daß aus den vereinigten Glycerinphasen eine Fettsäurephase gebildet wird, welche verestert und dem anderen Reaktionsgemisch, in dem die weitere Umesterung vorgenommen wird, zugeführt wird.

8. Verfahren nach einem oder mehreren der Ansprüche 1 bis 7, dadurch gekennzeichnet, daß als Alkohol für die Umesterung und die Veresterung Methanol oder Ethanol eingesetzt werden.

## Claims

1. A method for the preparation of fatty acid alkyl esters by transesterification, in particular catalytic transesterification, of triglycerides, wherein from a reaction mixture, in which the transesterification is carried out, an ester phase and a glycerol phase containing fatty acids, fatty acid salts and/or other fatty acid compounds, are formed, which are separated from each other, characterized in that the fatty acids, the fatty acid salts and/or other fatty acid compounds are separated from the glycerol phase, esterified with an alcohol from the group consisting of methanol, ethanol, propanol, 2-propanol, butanol, 2-butanol, pentanol, hexanol, heptanol and octanol, and recycled to a different reaction mixture, in which a further transesterification is carried out.

2. A method according to claim 1, characterized in that the esterification is carried out with an acidic catalyst.

3. A method according to claim 2, characterized in that the raw product obtained after esterification with an acid catalyst is added without further treatment to said different reaction mixture.

4. A method according to one or more of claims 1 to 3, characterized in that the transesterification is carried out under alkali catalysis.

5. A method according to one or more of claims 1 to 4, characterized in that the esterified fatty acids, fatty acid salts and/or other fatty acid compounds are added to said different reaction mixture at a point at which the further transesterification is essentially completed, but the ester phase and the glycerol phase of said further transesterification have not yet separated from each other.

6. A method according to one or more of claims 1 to 5, characterized in that the transesterification is carried out in various sequential stages, in each stage being formed a fatty acid phase which is esterified and added to a subsequent transesterification stage.

7. A method according to one or more of claims 1 to 5, characterized in that the transesterification is carried out in various sequential stages and that the glycerol phases produced in each of the stages are separated and combined, and in that from the combined glycerol phases a fatty acid phase is formed, which is esterified and fed to the other reaction mixture, in which the further transesterification is carried out.

8. A method according to one or more of claims 1 to 7, characterized in that methanol or ethanol is employed as alcohol for the transesterification and the esterification.

## Revendications

1. Procédé de préparation d'esters alkyliques d'acides gras par transestérification, plus particulièrement, par transestérification catalytique, de triglycérides, conformément auquel au départ d'un mélange réactionnel, dans lequel on entreprend la transestérification, on forme une phase d'ester et une phase de glycérine contenant des acides gras, des sels d'acides gras et/ou d'autres composés d'acides gras, qui sont séparés les uns des autres, caractérisé en ce que l'on sépare les acides gras, les sels d'acides gras et/ou les autres composés d'acides gras de la phase de glycérine, on les estérifie avec un alcool du groupe méthanol, éthanol, propanol, i-propanol, butanol, sec-butanol, pentanol, hexanol, heptanol et octanol et on les recycle dans un autre mélange réactionnel dans lequel on entreprend une transestérification supplémentaire.

2. Procédé suivant la revendication 1, caractérisé en ce que l'on entreprend l'estérification avec un catalyseur acide.

3. Procédé suivant la revendication 2, caractérisé en ce que l'on ajoute le produit brut obtenu après l'estérification avec un catalyseur acide sans autre traitement à l'autre mélange réactionnel.

4. Procédé suivant une ou plusieurs des revendications 1 à 3, caractérisé en ce que l'on entreprend la transestérification sous catalyse alcaline.

5. Procédé suivant une ou plusieurs des revendications 1 à 4, caractérisé en ce que l'on ajoute les acides gras, les sels d'acides gras et/ou les autres composés d'acides gras, estérifiés, à l'autre mélange réactionnel, à un moment auquel la transestérification supplémentaire est essentiellement terminée mais cependant auquel la phase d'ester et la phase de glycérine de l'autre transestérification ne sont pas encore mutuellement séparées.

6. Procédé suivant une ou plusieurs des revendications 1 à 5, caractérisé en ce que l'on entreprend la transestérification en plusieurs étapes successives, où, dans chaque étape, se forme une phase d'acide gras que l'on estérifie et envoie à une étape de transestérification subséquente.

7. Procédé suivant une ou plusieurs des revendications 1 à 5, caractérisé en ce que l'on entreprend la transestérification en plusieurs étapes successives et on sépare et réunit les phases de glycérine qui se présentent à chaque fois dans les étapes et, à partir des phases de glycérine réunies, on forme une phase d'acide gras que l'on estérifie et que l'on envoie à l'autre mélange réactionnel dans lequel on entreprend la transestérification supplémentaire.

8. Procédé suivant une ou plusieurs des revendications 1 à 7, caractérisé en ce que l'on utilise le méthanol ou l'éthanol, à titre d'alcool pour la transestérification et l'estérification.
